# EUROPEAN PATENT APPLICATION

(11) **EP 4 556 075 A1**
(43) Date of publication of application: **21.05.2025**
(21) Application number: 23839680.8
(22) Date of filing: 13.07.2023
(51) Int. Cl.: A61Q 1/02, A61Q 1/10, A61Q 5/06, C09D 11/17, A45D 34/04, A61K 8/81

(54) **MARKER COMPOSITION FOR SKIN, AND APPLICATION TOOL FOR SAME**

(30) Priority: 13.07.2022 JP 2022112309
(71) Applicant: MITSUBISHI PENCIL COMPANY, LIMITED, Tokyo 140-8537 (JP)
(72) Inventor: INOUE Kensuke, Fujioka-shi, Gunma 375-8501 (JP)
(74) Representative: Vossius & Partner Patentanwälte Rechtsanwälte mbB
(86) International application number: PCT/JP2023/025881
(87) International publication number: WO 2024/014508

(57) **Abstract**

Provided are a bright marker composition for skin and an application tool for the composition. The composition can prevent color separation using a dye and a flaky pigment with good color development and also prevent falling of the flaky pigment and absorption of the dye into the skin or the like, and can be applied easily. The marker composition for skin includes at least 0.95 to 4.75 mass% of an acid dye, an aminoalkyl (meth)acrylate copolymer, a water-soluble organic solvent, a flaky pigment, and water, wherein a pH is less than 7.0, the aminoalkyl (meth) acrylate copolymer is a copolymer polymerized from monomers, each of the monomers being a monomer selected from Group A and a monomer selected from Group B, and a ratio of the monomer of Group B is 2 to 15 mass% in the copolymer: Group A: ethyl (meth)acrylate, methyl (meth)acrylate, n-propyl (meth)acrylate, and the like; and Group **B:** 2-(dimethylamino)ethyl (meth)acrylate, 2-(diethylamino)ethyl (meth)acrylate, and 3-(dimethylamino)propyl (meth)acrylate.

## Description

### Technical Field

The present specification relates to a bright marker composition for skin and an application tool for the composition. The composition, while a dye of good color development and a flaky pigment are contained, can prevent color separation, falling of the flaky pigment and absorption of the dye into the skin or the like, and can be applied easily.

### Background Art

Examples of marker compositions for skin known in the art include:
(1) an ink composition for skin marking for directly marking the skin of a human body, the ink composition including: at least one component of ethyl alcohol, isopropyl alcohol, n-propyl alcohol, water, or the like as a solvent; at least one component of glycerin, propylene glycol, polyoxyethylene or a derivative thereof, or polyoxypropylene or a derivative thereof as a modifier; and a less-toxic food dye or the like (see Patent Document 1, for example);
(2) an ink composition for skin marking for directly marking the skin of a human body, the ink composition including: at least one or two or more components of ethyl alcohol, isopropyl alcohol, n-propyl alcohol, water, or the like as a solvent (s) ; at least one or two or more components of glycerin, propylene glycol, polyoxyethylene or a derivative thereof, or polyoxypropylene or a derivative thereof as a modifier (s) ; and one or two or more dyes of basic dyes (see Patent Document 2, for example) ;
(3) a makeup cosmetic containing an emulsifier-free polymerized resin emulsion (see Patent Document 3, for example);
(4) a makeup cosmetic containing a copolymer emulsion obtained from one or two or more acrylic acid ester monomers having 4 to 18 carbon atoms in the ester moiety and one or two or more methacrylic acid ester monomers (see Patent Document 4, for example);
(5) an aqueous coating composition for use as a face paint, a body paint, or the like, containing an aqueous isoprene rubber latex having an isoprene rubber component and a colorant (see Patent Document 5, for example); and
(6) a marker composition for skin containing 1 to 5 mass% of an acid dye, an aminoalkyl (meth)acrylate copolymer, a water-soluble organic solvent, and water, and having a pH of less than 7.0 (see Patent Document 6, for example).
   To impart brightness to a marker ink or a cosmetic not only for skin but also for other purposes, it is conceivable to add a pigment having brightness. The pigment having brightness has a large particle diameter and is likely to be precipitated and aggregated, and due to its shape, it is extremely difficult to redisperse the pigment when the a-b planes are brought into close contact with each other and aggregated, and therefore, there has been devised improvement to prevent precipitation and aggregation. As a countermeasure against such a problem, the applicant of the present invention has completed the following related art.
(7) Crystalline cellulose is added as a thickener, and a flaky pigment having a plurality of different particle diameters is used to prevent aggregation. Further, the ink composition is directly stored in a storage portion, and a stirring bar is enclosed therein to facilitate redispersion (see Patent Document 7, for example).
(8) The surface of a flaky pigment is covered with a water-insoluble substance, and the flaky pigment is dispersed in water to prevent aggregation. This precondition is also storage of the composition in a direct liquid state (see Patent Document 8, for example).

The ink compositions for skin marking of Patent Documents 1 and 2 described above include a "less-toxic food dye (Japanese Pharmacopoeia dye)" or a "basic dye", which can be used for marking during surgical operations, marking on foods, and the like. These inks firmly adhere to the skin or the like during use and can be easily removed, and are harmless even if they are absorbed by a body through the skin or the like and enter the body. However, although these ink compositions are non-toxic even if they enter the body by absorption through the skin or the like, the designs of these ink compositions do not encompass prevention of the ink from entering the body. Therefore, further improvement has been desired.

In addition, Patent Documents 3 and 4 describe compositions that can be used for body paints. Their approaches include the use of a pigment to prevent absorption into the skin. In these documents, the compositions use an emulsion resin for film formation, especially an acrylic emulsion resin, which can provide good film formation properties. These films have good properties, such as rubbing resistance and good water resistance, and thus have excellent "makeup durability". However, these compositions are obtained using mainly an iron oxide pigment which has a specific gravity greatly different from the emulsion, and this difference is likely to cause what is called color separation and problems, such as poor stability.

Patent Document 5 above describes use of a film-forming synthetic isoprene rubber latex to eliminate uncomfortable feelings, such as a "tight feeling" of the skin, and also exemplify dyes as colorants. Patent Document 5 above describes that the use of a synthetic isoprene rubber latex allows excellent elasticity and flexibility: however, the rubber film shrinks, and the shrinkage causes a "feeling of being pulled" and is also presumed to have poor weather resistance against direct sunlight and the like.

Further, in the above-described Patent Document 6 filed by the applicant of the present invention, the problems of Patent Documents 1 to 5 have been solved, however, it was not intended to stably contain a flaky (bright) pigment. In Patent Documents 7 and 8 also filed by the applicant of the present invention, it is assumed that the ink composition is stored in a direct-liquid type ink composition storage portion, that is, the ink composition and a stirring bar are enclosed in the storage portion, and writing (application) is performed only after the bright pigment is uniformly redispersed by shaking the writing instrument (application tool) before use.

### Citation List

### Patent Document

Patent Document 1: JP S55-137175 A (Claims, Description, etc.)
Patent Document 2: JP S58-154772 A (Claims, Description, etc.)
Patent Document **3:** JP S54-49338 A (Claims, Description, etc.)
Patent Document **4:** JP S54-151139 A (Claims, Description, etc.)
Patent Document 5: JP 2019-2002 A (Claims, Description, etc.)
Patent Document **6:** WO 2022/091971A (Claims, Description, etc.)
Patent Document 7: JP 2011-84661 A (Claims, Description, etc.)
Patent Document **8:** JP 2013-124360 A (Claims, Description, etc.)

### Summary of Invention

### Technical Problem

The present disclosure has been made in view of the above-described problems, current state, and the like of the related art, and an object of the present disclosure is to provide a bright marker composition for skin, while a dye of good color development and a flaky pigment are contained, that can prevent color separation, falling of the flaky pigment and absorption of the dye to the skin and the like, can be easily applied without a preliminary operation such as shaking, and has excellent visibility of a drawn line regardless of the color of the skin, and an application tool thereof.

### Solution to Problem

As a result of intensive studies in view of the above problems and the like of the related art, the disclosers of the present invention surprisingly found that an intended composition can be obtained only by adding a flaky pigment to the marker composition for skin of Patent Document 6, in which the marker composition for skin contains at least a predetermined amount of an acid dye, a specific copolymer, a water-soluble organic solvent, and water and having a pH of less than a specific value, and the composition can be stably discharged with a batting type writing instrument (application tool), thereby completing the present disclosure.

That is, a marker composition for skin of the present disclosure contains at least 0.95 to 4.75 mass% of an acid dye, an aminoalkyl (meth) acrylate copolymer, a water-soluble organic solvent, a flaky pigment, and water, wherein a pH is less than 7.0, the aminoalkyl (meth) acrylate copolymer is a copolymer polymerized from monomers, each of the monomers being a monomer selected from Group A and a monomer selected from Group B shown below, and a ratio of the monomer of Group B is 2 to 15 mass% in the copolymer:
Group A:
   ethyl (meth) acrylate, methyl (meth) acrylate, n-propyl (meth) acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate; and
Group B:
   2-(dimethylamino)ethyl (meth)acrylate, 2-(diethylamino)ethyl (meth)acrylate, and 3-(dimethylamino)propyl (meth)acrylate.

The acid dye is preferably a legally-permitted cosmetic dye. Here, the "legally-permitted cosmetic dye" refers to an organic synthetic dye that can be used in pharmaceutical products, quasi-pharmaceutical products and cosmetics as defined by the Ministry of Health, Labour and Welfare in 1996 and is classified into Group I (11 types), Group II (47 types), and Group III (25 types), 83 types in total.

The water-soluble organic solvent is preferably a lower alcohol having 3 or less carbons.

In the present disclosure, it is preferable that the cosmetic amount is a flaky pigment having an average particle diameter of 1 to 50 µm. In the present disclosure, it is preferable that the reservoir of the batting type application tool has a batting porosity of 88 to 96%, and it is preferable that the fibers of the fiber bundle of the application tool have a denier number of 2 to **5.** The denier number is the number of grams (weight) of the fiber of 9000 m, and the thicker the fiber, the larger the numerical value. The application tool formed of such thick fibers has a large gap between the fibers from the rear end of the application tool to the front end of the application tool, and the gap has many linear portions from the rear end of the application tool to the front end of the application tool.

### Advantageous Effects of Invention

The present disclosure can provide a bright marker composition for skin that can prevent color separation using a dye and a flaky pigment with good color development, can prevent falling of the flaky pigment and absorption of the dye into the skin or the like, can be easily applied with a batting type application tool, and has excellent visibility of drawn lines regardless of the color of the skin.

The object and effects of the present disclosure can be recognized and obtained especially using the components and combinations indicated in the claims. Both general explanation described above and detailed explanation described below are exemplary and explanatory and do not limit the present disclosure described in the claims.

### Description of Embodiments

Embodiments of the present disclosure will be described below in detail. However, it should be noted that the technical scope of the present disclosure is not limited to the embodiments detailed below and includes the invention described in the claims and equivalents thereof.

A marker composition for skin of the present disclosure includes at least 0.95 to 4.75 mass% of an acid dye, an aminoalkyl (meth)acrylate copolymer, a water-soluble organic solvent, a flaky pigment, and water, wherein a pH is less than 7.0, the aminoalkyl (meth) acrylate copolymer is a copolymer polymerized from monomers, each of the monomers being a monomer selected from Group A and a monomer selected from Group B, and a ratio of the monomer of Group B is 2 to 15 mass% in the copolymer:
Group **A:**
   ethyl (meth) acrylate, methyl (meth) acrylate, n-propyl (meth) acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate; and
Group B:
   2-(dimethylamino)ethyl (meth)acrylate, 2-(diethylamino)ethyl (meth)acrylate, and 3-(dimethylamino)propyl (meth)acrylate.

Examples of the acid dye used in the present disclosure typically include acid dyes, and preferably, at least one of acid dyes among legally-permitted cosmetic dyes can be used.

Specific examples of the acid dye include at least one (or a mixture of each one or two or more thereof) of Red No. 2 (C.I. 16185), Red No. 3 (C.I. 45430), Red No. 102 (C.I. 16255), Red No. 104 (1) (C.I. 45410), Red No. 105 (1) (C.I. 45440), Red No. 106 (C.I. 45100), Yellow No. 4 (C.I. 19140), Yellow No. 5 (C.I. 15985), Green No. 3 (C.I. 42053), Blue No. 1 (C.I. 42090), Blue No. 2 (C.I. 73015), Red No. 201 (C.I. 15850), Red No. 206 (C.I. 15630), Red No. 227 (C.I. 17200), Red No. 230 (C.I. 45380), Red No. 231 (C.I. 45410), Red No. 232 (C.I. 45440), Orange No. 205 (C.I. 15510), Orange No. 207 (C.I. 45425), Yellow No. 202 (1) and (2) (C.I. 45350) , Yellow No. 203 (C.I. 47005) , Green No. 201 (C.I. 61570), Green No. 204 (C.I. 59040), Green No. 205 (C.I. 42095), Blue No. 202 (C.I. 42052), Blue No. 203 (C.I. 42052), Blue No. 205 (C.I. 42090), Brown No. 2011 (C.I. 20170) , Red No. 401 (C.I. 45190) , Red No. 502 (C.I. 16155), Red No. 503 (C.I. 16150), Red No. 504 (C.I. 14700), Red No. 506 (C.I. 15620), Orange No. 403 (C.I. 12100), Orange No. 402 (C.I. 14600), Yellow No. 402 (C.I. 18950), Yellow No. 403 (1) (C.I. 10316), Yellow No. 406 (C.I. 13065), Yellow No. 407 (C.I. 18820), Green No. 401 (C.I. 10020) , Green No. 402 (C.I. 42085), Purple No. 401 (C.I. 60730), and Black No. 401 (C.I. 20470).

Among these acid dyes, combining, for example, a red dye, a yellow dye, a blue dye, or the like enables color matching to yellow to brown to black and can prepare any color.

The (total) content of these acid dyes is desirably 0.95 to 4.75 mass% (hereinafter, "mass%" is referred to simply as "%") and preferably 1 to 3% relative to the total amount of the marker composition for skin without the flaky pigment.

When the content of the acid dye is less than 0.95%, sufficient color-developing properties are not obtained, and when the content of the acid dye is more than 4.75%, sufficient water resistance is not obtained, and the absorption of the dye into the skin or the like is affected, which is not preferable.

The aminoalkyl (meth) acrylate copolymer used in the present disclosure is a component to prevent color separation despite the use of an acid dye with good color development and also to prevent absorption of the dye into the skin or the like, and examples include at least one of a copolymer of a 2-(dimethylamino)ethyl methacrylate, 2-ethylhexyl acrylate and ethyl methacrylate and a copolymer of 2-(dimethylamino)ethyl methacrylate, methyl methacrylate and n-butyl methacrylate.

The aminoalkyl (meth)acrylate copolymer of the present disclosure is a copolymer polymerized from at least one monomer selected from Group A and at least one monomer selected from Group B below. From the viewpoints of water resistance and absorption of the dye into the skin or the like, the ratio of the amine monomer of group B below needs to be 2 to 15% in the copolymer (monomer of Group A + monomer of Group B + polymerization initiator and the like described later), and desirably, the amine monomer of Group B is contained in preferably 8 to 15% in the copolymer. When the content of this amine monomer is too high, that is, more than 15%, the composition tends to easily flow with water. On the other hand, if the content of this amine monomer is less than 2%, it would affect the absorption of the dye into the skin or the like and thus is not preferred.
Group A:
   ethyl (meth) acrylate, methyl (meth) acrylate, n-propyl (meth) acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate; and
Group **B:**
   2-(dimethylamino)ethyl (meth)acrylate, 2-(diethylamino)ethyl (meth)acrylate, and 3-(dimethylamino)propyl (meth)acrylate.

The wording " (meth) acrylate" represents "acrylate and/or methacrylate" in group A above and the like. Particularly preferably, ethyl (meth)acrylate, which further improves adhesion of drawn lines to the skin or the like, is desirably used.

In group B above, particularly preferably, 2-(dimethylamino)ethyl methacrylate is desirably used.

For the aminoalkyl (meth)acrylate copolymer used in the present disclosure, a commercially available product can be used if available, and preferably, an aminoalkyl (meth) acrylate copolymer obtained by the following production method is desirably used.

The aminoalkyl (meth) acrylate copolymer used in the present disclosure can be produced by the procedures, for example, dissolving mixed monomers containing at least one monomer selected from group A above and at least one monomer selected from group B above in a solvent, such as n-propanol, and performing solution polymerization using azobisisobutyronitrile or the like as a polymerization initiator or further using a reducing agent in the polymerization initiator in combination, and if necessary further using a polymerizable surfactant.

In the present disclosure, for the content of the monomer of the Group B among the components of the aminoalkyl (meth) acrylate copolymer, the above preferred ratio of the content is desirable from the viewpoints such as color-developing properties, weather resistance, and stability, and the above content (2 to 15%) is set from the viewpoints such as water resistance and absorption to the skin or the like.

In the present disclosure, the aminoalkyl (meth) acrylate copolymer used in the present disclosure with a resin solid content of 10 to 35% is obtained in the preferred aspect, specifically, by dissolving the mixed monomers containing at least the monomer of group A above and the monomer of group B above in n-propanol or the like and performing solution polymerization.

The aminoalkyl (meth) acrylate copolymer obtained is useful for adhesion and water resistance.

The content of the aminoalkyl (meth)acrylate copolymer used is preferably 10 to 35% in terms of resin solid content relative to the total amount of the marker composition for skin from the viewpoints of highly achieving both fixing property and water resistance.

Examples of the water-soluble organic solvent used in the present disclosure include lower alcohols having 5 or less carbons. In particular, such a lower alcohol can reduce the solubility of the acid dye and the aminoalkyl (meth)acrylate copolymer to be obtained, and the viscosity of the composition containing these. This is preferred for allowing the composition to flow out when the composition is filled into an application tool and used. In addition, such a lower alcohol is also preferred for the drying property of the film after application. Specific examples of the water-soluble organic solvent include at least one (each alone or a mixture of two or more) of methyl alcohol (methanol) , ethyl alcohol (ethanol), n-propyl alcohol, isopropyl alcohol, n-butyl alcohol, sec-butyl alcohol, tert-butyl alcohol, isobutyl alcohol, pentyl alcohol, ethylene glycol, and propylene glycol. Preferably, a lower alcohol having 3 or less carbons, particularly ethyl alcohol (ethanol), n-propyl alcohol, or isopropyl alcohol, is desirably used from the viewpoints of safety and ease of handling.

Desirably, the content of the water-soluble organic solvent used is preferably 20 to 80%, more preferably 20 to 70% relative to the total amount of the marker composition for skin from the viewpoints of the solubility of the aminoalkyl (meth)acrylate copolymer to be obtained and the solubility of the acid dye.

Setting the content of this water-soluble organic solvent to 20% or more makes it possible to improve the solubility of the aminoalkyl (meth) acrylate copolymer to be obtained and provide a stable solution, and in addition, a slight antiseptic effect is exhibited. On the other hand, setting the content of this water-soluble organic solvent to 80% or less makes it possible to further improve the solubility of the acid dye and exhibit effects in terms of color-developing properties and water resistance.

The flaky pigment used in the present invention can be adopted without particular limitation. Examples of the flaky pigment include at least one (or a mixture of each one or two or more thereof, the same applies hereinafter) mica, mica titanium, carmine coated mica titanium, chromium oxide coated mica titanium, iron oxide coated mica titanium, iron oxide-carmine coated mica titanium, iron oxide-Prussian blue coated mica titanium, blue-coated mica titanium, Prussian blue coated mica titanium, red iron oxide coated mica, red iron oxide coated mica titanium, red iron oxide-carmine coated mica titanium, red iron oxide coated mica titanium, red iron oxide-iron oxide coated mica titanium, red iron oxide-Prussian blue coated mica titanium, red iron oxide-iron oxide-Prussian blue coated mica titanium, and bright pigments obtained by coating glass flakes or massive flakes as a base material with metal or metal oxide, for example, commercially available aluminum flake pigments, silica-coated aluminum pigments subjected to silica coating, flaky (scaly) pigments such as metal vapor deposition resin pigments. Of these, examples of the flaky pigment include commercially available aluminum flake pigments, metal oxide-coated glass flakes (trade name "METASHINE"; manufactured by Nippon Sheet Glass Co., Ltd. or the like) , and metal oxide-coated mica (trade name "Lumina" or the like; manufactured by Nagase & Co., Ltd.). Examples of the silica-coated aluminum pigment include trade name "Metallics" series (manufactured by Toyo Aluminium K.K.) and trade name "Visionaire" series (manufactured by ECKART GmbH).

As the flaky pigment used in the present disclosure, a silica-coated aluminum pigment is particularly preferable.

For water serving as a solvent used in the present disclosure, distilled water, ion-exchanged water, purified water, pure water, ultrapure water, or the like can be used. From the viewpoints of the solubility of the aminoalkyl (meth) acrylate copolymer and the solubility of the acid dye, desirably, the amount of water is preferably 10 to 35%, more preferably 15 to 30% relative to the total amount of the marker composition for skin.

The marker composition for skin of the present disclosure contains at least an acid dye, aminoalkyl (meth) acrylate copolymer, a water-soluble organic solvent, a flaky pigment, and water, while a pH adjuster, a surfactant, a viscosity modifier, or the like can be appropriately used as necessary from the viewpoints of further improving dispersibility and stability of each dissolved component.

The marker composition for skin of the present disclosure preferably has a viscosity of 10 to 50 mPa·s at a temperature of 25°C as measured with a cone-plate viscometer, with a standard cone rotor of a cone angle of 1°34', a cone radius of 24 mm, and a shear rate of 76.6 s⁻¹, TVE-25L manufactured by Toki Sangyo Co., Ltd., from the viewpoints of stability and applicability.

A more preferred range in the above viscosity range may vary according to the types of blended components of the marker composition for skin, but is desirably more preferably 10 to 45 mPa·s.

With a viscosity value of 10 mPa·s or higher, the marker composition can be applied to the skin or the like without causing liquid dripping, while with a viscosity value of 45 mPa·s or lower, the marker composition can be smoothly applied to the skin.

In addition, the viscosity can be adjusted to the above range by suitably combining the amount of each component, such as the acid dye, the aminoalkyl (meth)acrylate copolymer, water, and viscosity modifier if necessary.

The pH of the marker composition for skin of the present disclosure is adjusted to lower than 7.0 to improve coloring properties, prevent skin irritation, and stabilize dissolution, and desirably, the pH is preferably adjusted to 4 or higher and lower than 7.

The marker composition for skin with a pH of 7 or higher may reduce dyeing affinity to the skin, and the marker composition for skin with a pH lower than 4 may cause skin irritation.

In the present disclosure, the pH can be adjusted using a pH adjuster, including an organic acid, such as malic acid, citric acid, or glycolic acid; an inorganic acid or its salt, such as 5 M hydrochloric acid; or aminomethyl propanol.

In the present disclosure, when a pH adjusting agent is used, more preferably, the blending ratio of the aminoalkyl (meth)acrylate copolymer to the pH adjuster in terms of mass ratio is desirably 15:5 to 15:1 and particularly preferably, desirably 15:3 to 15:1.

With the ratio of 15:3 to 15:1, the marker composition for skin has low irritation to the skin in a weakly acidic region where the pH is lower than 7.0.

The marker composition for skin of the present disclosure can be prepared by a usual method and can be produced by blending each component, such as at least one of the acid dyes, the aminoalkyl (meth) acrylate copolymer, water, and the water-soluble organic solvent, in the range of each content described above and uniformly stirring and mixing them.

For example, the marker composition for skin without a flaky pigment can be prepared by stirring the acid dye and a solvent, such as water, with a commonly used disperser or the like until the mixture becomes uniform, then mixing the aminoalkyl (meth)acrylate copolymer, thereafter adding a pH adjuster, a viscosity modifier, or the like as necessary, and stirring with a homomixer or the like until the mixture becomes uniform with a disper or the like.

The flaky pigment is added at the end, and the composition can be prepared by stirring until the flaky pigment is uniformly seen with a commonly used disper or the like. The amount of the composition without a flaky pigment is adjusted to 92.5 to 98.0 parts by weight, the amount of the flaky pigment is adjusted to 2.0 to 7.5 parts by weight, and the total amount is adjusted to 100 parts by weight. The flaky pigment is preferably a flat pigment having an average particle diameter of 1 to 50 µm. When the average particle diameter is less than 1 µm, it is difficult to obtain sufficient brightness, and when the average particle diameter exceeds 50 µm, the liquid outflow amount decreases.

When the marker composition for skin of the present invention configured as described above is provided for use, the application tool is not particularly limited as long as it is an application tool that can apply the composition to a skin portion (skin) such as an acid dye, a face surface, or a body surface and dye the skin to easily form a drawing or the like on the skin portion. For example, an application tool for skin such as a brush attached container type, a spatula attached container type, a bottle type, a tube type, or a batting type application tool can be used. The shape, the structure, and the like of the application tool for skin to be used are not particularly limited, however, as a characteristic of the composition of the present invention, a batting type application tool can be used although a flaky pigment having an average particle diameter of large particles of 1 to 50 µm is contained.

The marker composition for skin of the present invention contains at least 0.95 to 4.75 mass% of an acid dye, an aminoalkyl (meth)acrylate copolymer polymerized from a combination of specific monomers and having a ratio of the monomers within a predetermined range, a water-soluble organic solvent, a flaky pigment, and water, wherein the pH of the marker composition for skin is less than 7.0. When the composition is applied to a desired skin portion to be applied using a batting type application tool without shaking or the like, good color development is obtained without color separation, the flaky pigment does not fall off unintentionally from the skin, absorption of the dye into the skin or the like can be prevented, and a beautiful finish can be provided.

It is desirable that in the application tool including the marker composition for skin of the present invention, the composition is impregnated into a batting type storage portion having a porosity of 88 to 96%. Setting the porosity to 88% or more makes it possible to discharge the flaky pigment, and on the other hand, setting the porosity to 96% or less makes it possible to prevent dripping, which enables stable discharge. The porosity in the present invention means a value obtained by subtracting the fiber proportion in the volume of the batting.

In the application tool of the present invention, the application portion preferably has a fiber bundle core formed of fibers of 2 to 5 denier, and further, the fiber bundle core preferably has a porosity of 70 to 80%. When the fiber bundle core has a denier number of 2 or more and a porosity of 70% or more, the flaky pigment is more favorably discharged, and on the other hand, when the fiber bundle core has a denier of 5 or less and a porosity of 80% or less, dripping is prevented, and stable discharge is possible.

### Examples

Next, the present disclosure will be described in further detail with reference to production examples, examples, and comparative examples. The present disclosure is not limited by the following examples and the like.

Aminoalkyl (meth)acrylate copolymers used in Examples 1 to 3, Comparative Examples 1 to 7, and Reference Examples 1 and 2 were obtained by the following Production Examples 1 to 7. In the following, "part (s)" represents "part(s) by mass".

### Production Example 1

An aminoalkyl (meth)acrylate copolymer was produced by the following procedure using 18.3 parts of ethyl methacrylate, 4 parts of 2-(dimethylamino)ethyl methacrylate, 9.3 parts of 2-ethylhexyl acrylate, 1 part of azobisisobutyronitrile (V-601, available from FUJIFILM Wako Pure Chemical Corporation), and 67.4 parts of n-propyl alcohol.

Next, a 500-milliliter flask was equipped with a stirrer, a reflux condenser, a thermometer, and a nitrogen gas inlet tube and set in a warm water bath, and the above materials were placed in the flask. The internal temperature was raised to 80°C while nitrogen gas was introduced, and solution polymerization was performed. The polymerization was terminated after 6 hours of aging, and an aminoalkyl (meth)acrylate copolymer [2-(dimethylamino)ethyl methacrylate/2-ethylhexyl acrylate/ethyl methacrylate copolymer] was obtained.

### Production Example 2

In Production Example 1 above, 1 part of azobisisobutyronitrile (V-601, available from FUJIFILM Wako Pure Chemical Corporation) was changed to 1 part of (AIBN, available from FUJIFILM Wako Pure Chemical Corporation), and an aminoalkyl (meth)acrylate copolymer was obtained in the same manner as in Production Example 1 above.

### Production Example 3

An aminoalkyl (meth) acrylate copolymer was obtained in the same manner as in Production Example 1 above by changing 4 parts of 2-(dimethylamino)ethyl methacrylate of Production Example 1 to 2.4 parts, and 67.4 parts of n-propyl alcohol to 69 parts (total amount 100 parts).

### Production Example 4

An aminoalkyl (meth) acrylate copolymer was obtained in the same manner as in Production Example 1 above, except for changing the amounts in Production Example 1 above to 17 parts of ethyl methacrylate, 6 parts of 2-(dimethylamino)ethyl methacrylate, and 8.6 parts of 2-ethylhexyl acrylate (total amount 100 parts) .

### Production Example 5

An aminoalkyl (meth) acrylate copolymer was obtained in the same manner as in Production Example 1 above, except for changing the amounts in Production Example 1 above to 15.6 parts of ethyl methacrylate, 8 parts of 2-(dimethylamino)ethyl methacrylate, and 8 parts of 2-ethylhexyl acrylate (total amount 100 parts) .

### Production Example 6

An aminoalkyl (meth) acrylate copolymer was obtained in the same manner as in Production Example 1 above, except for changing the amounts in Production Example 1 above to 10 parts of ethyl methacrylate, 15 parts of 2-(dimethylamino)ethyl methacrylate, and 5 parts of 2-ethylhexyl acrylate (total amount 100 parts).

### Production Example 7

An aminoalkyl (meth) acrylate copolymer was obtained in the same manner as in Production Example 1 above, except for changing the amounts in Production Example 1 above to 6 parts of ethyl methacrylate, 21 parts of 2-(dimethylamino)ethyl methacrylate, and 3 parts of 2-ethylhexyl acrylate (total amount 100 parts).

Using the aminoalkyl (meth)acrylate copolymers of Production Examples 1 to 7 thus obtained, a composition without a flaky pigment was first obtained according to the formulation shown in Table 1 below, and then a target bright marker composition for skin was obtained with a blending proportion of 95% of the composition without a flaky pigment and 5% of a flaky pigment (bright pigment).

The resulting marker compositions for skin were evaluated for a viscosity value, a pH value, color-developing properties, water resistance, and water-resistant adhesion by the following methods. The results are shown in Table 1 below.

### Method of Measuring Viscosity

Each marker composition for skin obtained by the above method was measured for viscosity under conditions of a temperature of 25°C with a cone-plate viscometer, a standard cone rotor with a cone angle of 1°34', a cone radius of 24 mm, a shear rate of 76.6 s⁻¹, TVE-25L manufactured by Toki Sangyo Co., Ltd.

### Method of Measuring pH

Each marker composition for skin obtained by the above method was measured for pH at 25°C with a glass electrode pH meter.

### Method for Evaluating Discharge Performance and Color-Developing Property

Each resulting marker composition for skin was filled into an application tool equipped with a marker pen container (PC-IMR, application portion: nylon pen core (fiber bundle core: 2 denier, porosity: 80%), with batting storage having porosities of 82% and 94% in application liquid storage portion) manufactured by Mitsubishi Pencil Co., Ltd. and applied (a line was drawn on) to the back of a hand. Then, evaluation was performed for discharge performance and color-developing properties according to the following evaluation criteria.

Evaluation criteria for discharge performance:
A: The application liquid having brightness was discharged without any problem.
B: The application liquid was discharged, but the brightness was slightly inferior to that of the contained composition.
C: The application liquid was discharged, but the brightness was inferior to that of the contained composition.
D: The application liquid was not discharged, or even when it was able to be discharged, no brightness was observed.

Evaluation criteria for color-developing property:
A: Vibrant color development and brightness are observed.
B: Slight faintness is observed in color development, or brightness is slightly poor.
C: Faintness is observed in color development, or brightness is poor.
D: Remarkable- faintness is observed in color development, or no brightness is observed.

Method for evaluating water resistance and water-resistant adhesion Each obtained marker composition for skin was filled into an application tool equipped with an eyeliner/eyebrow container (PC-IM, application portion: nylon pen core (fiber bundle core: 2 denier, porosity: 80%), with batting storage having porosities of 82% and 94% in application liquid storage portion) manufactured by Mitsubishi Pencil Co., Ltd., and applied (a line was drawn on) to the back of a hand. Then, evaluation was performed for water resistance and water-resistant adhesion according to the following evaluation criteria.

Evaluation criteria for water resistance:
A: No change is observed in the drawn line with running water.
B: Slight change (color loss, falling of bright pigment) is observed in the drawn line with running water.
C: Significant change is observed in the drawn line with running water.
   - : Cannot be evaluated.

Evaluation criteria for water-resistant adhesion:
A: No change is observed in the drawn line when the line is rubbed with the pad of a finger after running water.
B: Slight change is observed in the drawn line when the line is rubbed with the pad of a finger after running water.
C: Change is observed in the drawn line when the line is rubbed with the pad of a finger after running water.
   - : Cannot be evaluated.

**[Table 1]**

| (Total amount 100 mass%) | | | | | | | | | | | | |
|---|---|---|---|---|---|---|---|---|---|---|---|---|
| | Examples | | | Comparative Example | | | | | | | Reference Example | |
| | 1 | 2 | 3 | 1 | 2 | 3 | 4 | 5 | 6 | 7 | 1 | 2 |
| Acid dye *1 | 3 | 3 | 3 | 6 | 3 | 3 | 3 | 3 | 3 | | 3 | 3 |
| Basic dye *2 | | | | | | | | | | 3 | | |
| Flaky pigment *3 | 5 | 5 | 5 | 5 | 5 | 5 | 5 | 5 | 5 | 5 | 5 | 5 |
| Aminoalkyl (meth)acrylate copolymer[Group A×Group B] Production Example No. | 1 | 2 | 3 | 1 | 1 | 4 | 5 | 6 | 7 | 1 | 1 | 1 |
| Content (solid content) | 50 (15) | 50 (15) | 50 (15) | 50 (15) | 50 (15) | 50 (15) | 50 (15) | 50 (15) | 50 (15) | 50 (15) | 50 (15) | 50 (15) |
| Ratio of Group B monomer (mass%) | 12.7 | 12.7 | 8.1 | 12.7 | 12.7 | 19 | 25.3 | 50 | 67.7 | 12.7 | 12.7 | 12.7 |
| pH adjuster (5M HCl) | 2.3 | 2.3 | 1.5 | 2.3 | 0 | 2.3 | 2.3 | 2.3 | 2.3 | 2.3 | 2.3 | 2.3 |
| n-Propyl alcohol | 30 | 30 | 30 | 30 | 30 | 30 | 30 | 30 | 30 | 30 | 30 | 30 |
| Water (purified water) | Balance | Balance | Balance | Balance | Balance | Balance | Balance | Balance | Balance | Balance | Balance | Balance |
| Viscosity (mPa·s) | 19.4 | 20 | 17.7 | 20.5 | *4 | 23 | 23.4 | 24 | 25.5 | *4 | 19.4 | 19.4 |
| pH | 5.4 | 5.4 | 5.8 | 4.8 | 8.2 | 6.7 | 6.8 | 7.2 | 7.5 | 5.4 | 5.4 | 5.4 |
| Batting porosity | 94 | 94 | 94 | 94 | 94 | 94 | 94 | 94 | 94 | 94 | 82 | 94 |
| Fiber bundle core denier no. | 2 | 2 | 2 | 2 | 2 | 2 | 2 | 2 | 2 | 2 | 2 | 0.5 |
| Discharge performance | A | A | A | A | - | A | A | A | A | - | C | C |
| Color-developing property | A | A | A | A | D | A | A | A | A | D | A | A |
| Water resistance | A | A | A | B | - | B | C | C | C | - | A | A |
| Water-resistant adhesion | A | A | A | A | - | C | C | C | C | - | A | A |

| | | | | | | | | | | | | |
|---|---|---|---|---|---|---|---|---|---|---|---|---|
| *1: Blue No. 1 (C.I. 42090) + Orange No. 205 (C.I. 15510) (mass ratio 1 : 1) *2: Basic Blue 99 *3: Visionaire Splendid Silver Sea (ECKART GmbH) (5 to 50 µm) *4: Dye is undissolved | | | | | | | | | | | | |

From the results in Table 1 above, it was found that Examples 1 to 3, which fall within the scope of the present invention, are superior in discharge performance, color-developing properties, water resistance, and water-resistant adhesion to Comparative Examples 1 to 7, which fall outside the scope of the present invention. In addition, when the nylon pen core was observed after use, it was found that the nylon pen core was not dyed, aggregation and discharge failure of the flaky pigment in the batting were not observed, and the drawing line density and color tone were stable. Further, in Reference Examples 1 and 2, since the marker composition for skin of Example 1 was mounted, color-developing properties, water resistance, and water-resistant adhesion were good, however, when the batting porosity of the application tool and the denier number of the fiber bundle core were out of preferable ranges, discharge performance was slightly poor.

### Industrial Applicability

There is provided a bright marker composition for skin that can be easily applied to a skin portion to which application of the composition is intended, such as a face surface and body without a preliminary operation. This bright marker composition for skin exhibits excellent color development without color separation and can also prevent absorption of the dye into the skin or the like, and can provide a beautiful finish. In addition, this marker composition for skin can also be used as an eyebrow cosmetic or a temporary hair dye.

## Claims

1. A marker composition for skin, the composition comprising at least 0.95 to 4.75 mass% of an acid dye, an aminoalkyl (meth)acrylate copolymer, a water-soluble organic solvent, a flaky pigment, and water, wherein a pH is less than 7.0, the aminoalkyl (meth)acrylate copolymer is a copolymer polymerized from monomers, each of the monomers being a monomer selected from Group A and a monomer selected from Group B, and a ratio of the monomer of Group B is 2 to 15 mass% in the copolymer:
Group A:
ethyl (meth)acrylate, methyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate; and
Group **B:**
2-(dimethylamino)ethyl (meth)acrylate, 2-(diethylamino)ethyl (meth)acrylate, and 3-(dimethylamino)propyl (meth)acrylate.

2. The marker composition for skin according to claim 1, wherein the acid dye is a legally-permitted cosmetic dye.

3. The marker composition for skin according to claim 1 or 2, wherein the water-soluble organic solvent is a lower alcohol having 3 or less carbons.

4. An application tool comprising the marker composition for skin according to claim 1, wherein the marker composition for skin is impregnated into a batting type storage portion having a porosity of 88 to 96%.

5. The application tool comprising the marker composition for skin according to claim 4, the application tool having a fiber bundle core of 2 to 5 denier.
